# EUROPÄISCHE PATENTSCHRIFT

(11) **EP 3 790 536 B1**
(45) Veröffentlichungstag und Bekanntmachung des Hinweises auf die Patenterteilung: **22.04.2026**
(21) Anmeldenummer: 19722893.5
(22) Anmeldetag: 08.05.2019
(51) Int. Cl.: A61K 9/51, A61P 31/06, A61P 35/00, A61K 9/00, A61K 47/02, A61K 47/12, A61K 47/20, A61K 47/24, A61K 31/47

(54) **NANOCONTAINER FÜR DEN TRANSPORT UNPOLARER, LIPOPHILER SUBSTANZEN WIE ANTIBIOTIKA**
NANOCONTAINERS FOR TRANSPORTING NONPOLAR, LIPOPHILIC SUBSTANCES SUCH AS ANTIBIOTICS
NANORÉCIPIENT DESTINÉ AU TRANSPORT DE SUBSTANCES NON POLAIRES, LIPOPHILES, TELLES QUE DES ANTIBIOTIQUES

(30) Priorität: 09.05.2018 DE 102018003770
(43) Veröffentlichungstag der Anmeldung: 17.03.2021
(73) Patentinhaber: Karlsruher Institut für Technologie, 76131 Karlsruhe (DE); Forschungszentrum Borstel, 23845 Borstel (DE)
(72) Erfinder: FELDMANN, Claus, 76275 Ettlingen (DE); REIN, Viktor, 74239 Hardthausen (DE); SCHAIBLE, Ulrich, 22359 Hamburg (DE); REDINGER, Natalja, 22523 Hamburg (DE); HAGENS, Kristine, 23866 Nahe (DE)
(74) Vertreter: Müller-Boré & Partner Patentanwälte PartG mbB
(86) Internationale Anmeldenummer: PCT/EP2019/061820
(87) Internationale Veröffentlichungsnummer: WO 2019/215231

(56) Entgegenhaltungen:
- WO-A1-2012/071014
- WO-A2-2008/149096
- DE-A1- 102007 023 491

## Beschreibung

Die vorliegende Erfindung betrifft Nanocontainer, geeignet als neue Verabreichungsform unpolarer, lipophiler Verbindungen wie Antibiotika, insbesondere für die Behandlung von schwerwiegenden Infektionserkrankungen. Basierend auf einer einfachen, wasserbasierten Synthese ist eine breite Substanzpalette unpolarer, lipophiler Verbindungen mit breiter Wirkstoff- und Therapiebasis verfügbar. Zudem ist in einfacher Weise eine Kombination von Wirkstofffreisetzung mit optischer Detektion möglich. Die erfindungsgemäßen Nanocontainer vereinen damit Therapie (Wirkstofffreisetzung) und Diagnostik (optische Detektion von Nachweisreagentien).

Unpolare, lipophile Verbindungen, insbesondere Wirkstoffe, sind von einer effektiven klinischen Anwendung häufig ausgeschlossen, da diese nicht oder nur sehr schwer verabreicht werden können und/oder den Wirkort nur in unzureichender Konzentration erreichen. Dies ist insbesondere der Fall, wenn lipophile Verbindungen intravenös über die Blutbahn verabreicht werden oder wenn lipophile Verbindungen in wässriges Milieu eingebracht werden sollen (z.B. Lungenbläschen). Darüber hinaus ist die Zellaufnahme bzw. der Transport durch Membranen für unpolare, lipophile Verbindungen häufig gegenüber polaren, hydrophilen Verbindungen stark herabgesetzt.

Grundsätzlich steht eine ganze Reihe an, zum Teil schon lange bekannten, sehr wirksamen Antibiotika für die unterschiedlichste Anwendungsfälle und Therapien zur Verfügung. Allerdings müssen diese antibakteriellen Wirkstoffe nicht nur Granulom-Einkapselungen und die Membran der Wirtszellen, sondern gegebenenfalls auch lipidreiche Zellwände mykobakterieller Zellen durchdringen. Weitere Einschränkungen betreffen die geringe Permeabilität in Biofilmen sowie der schnelle Abbau unter physiologischen Bedingungen bei geringer Dosis, aber schweren Nebenwirkungen bei höherer Dosis. Zu geringe Dosen sind andererseits der wesentliche Grund für ein generelles Therapieversagen sowie die Hauptgefahr für bakterielle Multiresistenzen.

Vor diesem Hintergrund sind kostengünstige und effiziente Wirkstoffe und Verabreichungsformen erforderlich, um geringere Dosierungen und kürzere Behandlungsdauern zu erreichen und Nebenwirkungen zu vermeiden. Dies würde die Patientenzustimmung erhöhen und das Risiko therapeutischer Misserfolge und bakterieller Resistenzen minimieren. Nanomaterialien bieten generell bereits vielversprechende Strategien für Molekularbiologie und Medizin. So wurde deren Verwendung für neuartige Bildgebungstechniken oder die Tumortherapie in den letzten Jahren bereits intensiv untersucht. Für die Behandlung mit Wirkstoffen wie Antibiotika wären ebenso effiziente Transportsysteme zur Infiltration der Antibiotika in infizierte Zellen erforderlich. Derartige Transportsysteme sollten möglichst hohe Antibiotikagehalte aufweisen und zudem bioverträglich, leicht abbaubar und im besten Fall nach 2 bis 3 Tagen vollständig ausgeschieden sein. Derartige nanoskalige Transportsysteme wurden bislang wenig untersucht. Insbesondere wurden SiO₂- und Polymernanopartikel mit Antibiotika beschichtet oder beladen, deren Antibiotikagehalt bezogen auf die Gesamtmasse der Nanopartikel jedoch gering ist (d.h. Antibiotikabeladung < 10 Gew.-%). Das inerte Trägersystem stellt damit die Mehrheitskomponente dar (> 90 Gew.-%). Vom Trägersystem geht in der Regel keine Wirksamkeit aus. Das Trägersystem kann jedoch eine Reihe von Nachteilen mit sich bringen, die von Nebenwirkungen über toxische Effekte bis zu unzureichender Bioabbaubarkeit reichen können. Die meisten nanopartikulären Systeme beziehen sich darüber hinaus auf den Transport polarer, hydrophiler Antibiotika.

Als konkreter Anwendungsfall sei hier Tuberkulose (TB) genannt, die gemäß Weltgesundheitsorganisation (World Health Organization, WHO) zu den weltweit am weitesten verbreiteten Infektionskrankheiten mit hoher Mortalität gehört. So wurden 2013 etwa 9 Millionen TB-Erkrankungen mit 1,5 Millionen Todesfällen gemeldet. TB hat inzwischen wieder erhebliche Bedeutung in Verbindung mit HIV und infolge der besorgniserregenden multiresistenten (Multidrug Resistant, MDR) und extrem resistenten (Extrem Drug Resistant, XDR) Isolate gewonnen. Weiterhin ist davon auszugehen, dass etwa ein Drittel der Weltbevölkerung latent infiziert ist, wobei die Wahrscheinlichkeit eines akuten Verlaufs bei etwa 10% über die Lebenszeit liegt. Der pathogene Erreger, *M*. *tuberculosis* (*M.tb.*)*,* befindet sich als fakultativ interzellulärer Parasit in Makrophagen eingekapselt in Granulome. Vor diesem Hintergrund stellt insbesondere die latente Tuberkulose eine große Herausforderung dar. Geeignete unpolare, lipophile Wirkstoffe sind hier zum Beispiel Delamanid, Bedaquilin, Benzothiazon (BTZ), Amikazin, Clofazimin, Levofloxacin oder Pantoprazol.

Weitere Anwendungsfälle beziehen sich auf multiresistente Bakterien wie *Staphylococcus aureus, Klebsiella pneumoniae, Pseudomonas aeruginosa, Acinetobacter baumannii* oder verschiedene *Enterobacter-Typen,* die als "Krankenhauskeime" bekannt geworden sind und zu hohen Todesfallzahlen in Krankenhäusern führen.

Diese beiden Anwendungsfälle stehen stellvertretend für viele weitere bakterielle Erkrankungen und können jeweils als Massenmarkt bezeichnet werden, was die Relevanz überdeutlich macht. Neben neuen Antibiotika sind hier neue, effiziente Darreichungsformen von gleichbedeutender Aktualität und Relevanz. Das Patent WO 2012/071014 offenbart Kern-Hülle-Partikel, die jeweils umfassen: einen Kern, der ein hydrophobes Polymer mit einer anionischen Gruppe enthält; und eine Hülle, die Calciumphosphat enthält, wobei mindestens eines der in dem Calciumphosphat enthaltenen Calciumatome chemisch an eine funktionelle Gruppe gebunden ist, die von der anionischen Gruppe abgeleitet ist.

Vor diesem Hintergrund ist es die Aufgabe der vorliegenden Erfindung, eine bioverträgliche und leicht abbaubare Verabreichungsform für unpolare, lipophile Verbindungen mit sehr breitem Anwendungsspektrum, welche eine große Beladungsmenge an unpolarer, lipophiler Verbindung ermöglicht und gleichzeitig eine optische Erkennung zulässt und welche über eine sehr einfache Synthese zugänglich ist, sowie ein kostengünstiges und effizientes Verfahren zur Herstellung dieser Verabreichungsform bereitzustellen.

Diese Aufgabe wird durch die in den Ansprüchen gekennzeichneten Ausführungsformen gelöst.

Insbesondere wird erfindungsgemäß ein Nanocontainer gemäß Anspruch 1 bereitgestellt, umfassend eine Emulsion, umfassend mindestens eine unpolare, lipophile Verbindung, eingekapselt von einem biokompatiblen Tensid, welches mindestens eine polare Gruppe, ausgewählt aus einer Phosphatgruppe, einer Sulfatgruppe, einer Sulfonatgruppe oder einer Carboxylgruppe, aufweist, und
eine anorganische, biokompatible Hülle, welche die Emulsion umschließt,
wobei die polare Gruppe ionisch an die anorganische, biokompatible Hülle gebunden ist und dadurch das Tensid in Folge seiner Ausrichtung die Kavität der anorganischen Hülle lipophilisiert.

Der erfindungsgemäße Nanocontainer umfasst eine Emulsion, die mindestens eine unpolare, lipophile Verbindung umfasst. Die mindestens eine unpolare, lipophile Verbindung ist dabei von einem biokompatiblen Tensid eingekapselt, welches mindestens eine polare Gruppe, ausgewählt aus einer Phosphatgruppe, einer Sulfatgruppe, einer Sulfonatgruppe oder einer Carboxylgruppe, aufweist. Zudem weist das Tensid einen unpolaren, hydrophoben Rest auf. Der erfindungsgemäße Nanocontainer umfasst weiter eine anorganische, biokompatible Hülle, welche die Emulsion umschließt. Die polare Gruppe des biokompatiblen Tensids ist dabei ionisch an die anorganische, biokompatible Hülle gebunden, wodurch der unpolare Rest des Tensids in die Kavität der Hülle, d.h. in den von der Hülle umschlossenen (Hohl-)Raum, weist. Das Tensid befindet sich damit innerhalb der biokompatiblen Hülle, d.h. innerhalb der (Hohl-)Kugel. Durch diese Ausrichtung des Tensids wird die Kavität der anorganischen Hülle lipophilisiert. Die anorganische, biokompatible Hülle stabilisiert durch den Aufbau des erfindungsgemäßen Nanocontainers mechanisch die Emulsion, so dass diese als Transport- und Lagerform verfügbar gemacht wird. Erfindungsgemäß sind somit Tensid und unpolare, lipophile Verbindung innerhalb der (Hohl)-Kugel, d.h. innerhalb der Hülle, eingekapselt.

Erfindungsgemäß werden unter dem Begriff "unpolare, lipophile Verbindung" Verbindungen verstanden, die im Wesentlichen unlöslich in Wasser (beispielsweise < 0,01g/L) und sehr gut löslich (beispielsweise > 0,1 g/L) in Alkanen, wie beispielsweise Hexan und Dodecan, und/oder aromatischen Kohlenwasserstoffen, wie beispielsweise Toluol und Tocopherol, sind. Die Emulsion des erfindungsgemäßen Nanocontainers kann entweder eine oder auch mehrere unpolare, lipophile Verbindungen umfassen. Unpolare, lipophile Verbindungen sind erfindungsgemäß pharmazeutische aktive Wirkstoffe und/oder Nachweisreagenzien. Werden ein pharmazeutisch aktiver Wirkstoff und ein Nachweisreagens in Kombination eingesetzt, kann der erfindungsgemäße Nanocontainer vorteilhafterweise den Wirkstoff nach der Darreichung freisetzen und aufgrund des Nachweisreagens in z.B. Zellen, Geweben und Organen lokalisiert werden.

Unter dem Begriff "pharmazeutisch aktiver Wirkstoff" wird erfindungsgemäß ein Stoff verstanden, der als Mittel zur Heilung oder zur Verhütung menschlicher oder tierischer Krankheiten verwendet wird, sowie ein Stoff, der dazu bestimmt ist, im oder am menschlichen oder tierischen Körper zur Wiederherstellung, Besserung oder Beeinflussung der menschlichen oder tierischen Körperfunktionen angewandt zu werden.

Gemäß einer Ausführungsform der vorliegenden Erfindung ist der pharmazeutisch aktive Wirkstoff ausgewählt aus
der Gruppe der Antibiotika, bestehend aus Delamanid, Bedaquilin, Benzothiazon, Amikazin, Clofazimin, Levofloxacin, Ofloxacin, Rifampicin, Pantoprazol, Pyrimethamin, Trimethoprim, Sulfamethoxazol, Sulfadoxin, Novobiocin, Coumermycin, Clorobiocin, Metronidazol, Norfloxacin, Enoxacin, Ciprofloxacin, Ofloxacin, Levofloxacin, Moxifloxacin, Tigecyclin, Tetracyclin, Gentamicin, Kanamycin, Neomycin, Netilmicin, Streptomycin, Tobramycin, Chloramphenicol, Fusidinsäure, Cethromycin, Narbomycin, Telithromycin, Lincomycin, Daptomycin, Dalfopristin, Quinupristin, Azithromycin, Clarithromycin, Erythromycin, Roxithromycin, Linezolid, Doxycyclin, Minocyclin, Tetracyclin, Oxytetracyclin, Tigecyclin Imipenem, Meropenem, Ertapenem, Aztreonam, Benzylpenicillin, Phenoxymethylpenicillin, Piperacillin, Mezlocillin, Ampicillin, Amoxicillin, Flucloxacillin, Methicillin, Oxacillin, Clavulansäure, Sulbactam, Tazobactam, Sultamicillin, Teicoplanin, Vancomycin, Bacitracin, Colistin, Gramicidin, Polymyxin B, Tyrothricin und Teixobactin; oder
aus der Gruppe der Cytostatika, bestehend aus Cyclophosphamid, Mechlorethamin, Dacarbazine, Nitrosoureas, Temozolomid, Daunorubicin, Epirubicin, Idarubicin, Mitoxantron, Valrubicin, Paclitaxel, Docetaxel, Abraxan, Taxoter, Vorinostat, Romidepsin, Irinotecan, Topotecan, Etoposid, Teniposid, Tafluposid, Bortezomib, Erlotinib, Gefitinib, Imatinib, Vemurafenib, Vismodegib, Azacitidin, Azathioprin, Capecitabin, Cytarabin, Doxifluridin, Fluorouracil, Gemcitabin, Hydroxyurea, Vinblastin, Vincristin, Vindesin und Vinorelbine; oder
aus der Gruppe der Virostatika, bestehend aus Ancriviroc, Aplaviroc, Cenicriviroc, Enfuvirtid, Maraviroc, Vicriviroc, Amantadin, Rimantadin, Pleconaril, Idoxuridin, Aciclovir, Brivudin, Famciclovir, Penciclovir, Sorivudin, Valaciclovir, Cidofovir, Brincidofovir, Ganciclovir, Valganciclovir, Foscarnet, Ribavirin, Taribavirin, Filibuvir, Nesbuvir, Sofosbuvir, Tegobuvir, Favipiravir, Abacavir, Didanosin, Elvucitabin, Emtricitabin, Fosalvudintidoxil, Fozivudintidoxil, Stavudin, Zalcitabin, Zidovudin, Lamivudin, Lagociclovir, Tenofovir, Adefovir, Alamifovir, Clevudin, Entecavir, Pradefovir, Telbivudin, Delavirdin, Efavirenz, Emivirin, Etravirin, Lersivirin, Nevirapin, Rilpivirin, Amprenavir, Atazanavir, Brecanavir, Darunavir, Fosamprenavir, Indinavir, Lopinavir, Mozenavir, Nelfinavir, Ritonavir, Saquinavir, Tipranavir, Asunaprevir, Balapiravir, Boceprevir, Ciluprevir, Danoprevir, Daclatasvir, Narlaprevir, Telaprevir, Simeprevir, Vaniprevir, Rupintrivir, Elvitegravir, Dolutegravir, Raltegravir, Fomivirsen, Amenamevir, Bevirimat, Letermovir, Laninamivir, Oseltamivir, Peramivir und Zanamivir.

Gemäß einer Ausführungsform der vorliegenden Erfindung beträgt die Masse des pharmazeutisch aktiven Wirkstoffs mindestens 5 Gew.-%, bezogen auf die Gesamtmasse des Nanocontainers, bevorzugt mindestens 10 Gew.-%, besonders bevorzugt mindestens 15 Gew.-%, und am meisten bevorzugt mindestens 20 Gew.-%. Mit dem erfindungsgemäßen Nanocontainer lassen sich sogar unter Umständen Wirkstoffbeladungen pro Nanopartikel von 70 bis 95 Gew.-%, bezogen auf die Gesamtmasse des Nanocontainers, realisieren. Da vom Trägersystem des Nanocontainers, d.h. von den weiteren Bestandteilen außer dem Wirkstoff, üblicherweise keine pharmazeutische Wirksamkeit ausgeht, kann mit dem erfindungsgemäßen Nanocontainer aufgrund seines Aufbaus vorteilhafterweise eine große Beladungsmenge an Wirkstoff realisiert werden, so dass eine sehr hohe pharmazeutische Wirksamkeit pro verabreichter Nanocontainermenge erzielt werden kann.

Unter dem Begriff "Nachweisreagens" wird erfindungsgemäß ein Stoff bzw. eine Verbindung verstanden, der bzw. die nach Verabreichung im Körper detektiert/lokalisiert werden kann, beispielsweise optisch über Fluoreszenz im Falle eines Fluoreszenzfarbstoffs oder aber auch durch Röntgenabsorption oder magnetische Messungen.

Gemäß einer Ausführungsform der vorliegenden Erfindung ist das Nachweisreagens aus der Gruppe der Fluoreszenzfarbstoffe, beispielsweise aus Coumarin 6, Lumogen Rot, Fluoresceindiacetat, Oxonol, Nilrot und Fluoresceinisothiocyanat, ausgewählt.

Die mindestens eine unpolare, lipophile Verbindung ist in der Emulsion des erfindungsgemäßen Nanocontainers von einem biokompatiblen Tensid eingekapselt. Erfindungsgemäß wird unter dem Begriff "biokompatibel" verstanden, dass das Tensid keinen negativen Einfluss auf Lebewesen hat. Die Zertifizierung der Biokompatibilität kann beispielsweise über ISO 10993 1-20 erfolgen. Das biokompatible Tensid unterliegt erfindungsgemäß keiner besonderen Einschränkung, solange es mindestens eine polare Gruppe (polarer, hydrophiler Rest) aufweist, ausgewählt aus einer Phosphatgruppe, einer Sulfatgruppe, einer Sulfonatgruppe oder einer Carboxylgruppe. Zudem weist das Tensid einen unpolaren, hydrophoben Rest auf.

Erfindungsgemäß kann die Emulsion ein einziges Tensid oder ein Gemisch aus zwei oder mehr Tensiden umfassen. Gemäß einer bevorzugten Ausführungsform umfasst das Tensid ein Alkylphosphat, ein Alkylsulfat, ein Alkylsulfonat oder ein Alkylcarboxylat. Die Alkylgruppe weist dabei vorzugsweise jeweils von 5 bis 20 Kohlenstoffatome auf, besonders bevorzugt von 8 bis 15 Kohlenstoffatome, und am meisten bevorzugt von 10 bis 12 Kohlenstoffatome auf. Gemäß einer besonders bevorzugten Ausführungsform umfasst das Tensid Natrium- oder Kaliummonodecylphosphat, Natrium- oder Kaliumdodecylphosphat, Natrium- oder Kaliumtocopherolphosphat, Natrium- oder Kaliumdodecylsulfat, Natrium- oder Kaliumlaurinat oder Natrium- oder Kaliumcaprinat.

Der erfindungsgemäße Nanocontainer umfasst weiter eine anorganische, biokompatible Hülle, welche die Emulsion umschließt. Die mindestens eine polare Gruppe des biokompatiblen Tensids, welches sich in der Emulsion und deshalb innerhalb der Hülle befindet, ist dabei an die anorganische, biokompatible Hülle ionisch (von innen) gebunden, wodurch das Tensid in Folge seiner Ausrichtung (unpolarer Rest des Tensids weist in die Kavität der Hülle) die Kavität der anorganischen Hülle lipophilisiert, so dass die Emulsion von der Hülle mechanisch stabilisiert wird. Da die anorganische, biokompatible Hülle vorzugsweise ein Zeta-Potential bei pH 7 von mindestens -20 mV aufweist, gewährleistet sie eine hohe Ladungsstabilisierung, welche vorteilhafterweise die Agglomeration in wässriger Suspension wirkungsvoll unterbindet und gleichzeitig ein Redispergieren von Pulvern in Wasser bzw. ein Aerolisieren von Suspension und/oder Pulver erlaubt.

Gemäß der Ansprüche der vorliegenden Erfindung umfasst die anorganische, biokompatible Hülle ein Metallphosphat, ein Metallhydrogenphosphat, ein Metalldihydrogenphosphat, ein Metallsulfat oder ein Metallcarbonat, wobei das Metall (bzw. das Metallkation des Salzes) mindestens ein Metall (bzw. Metallkation), ausgewählt aus der Gruppe, bestehend aus Zr⁴⁺, ZrO²⁺, Mg²⁺, Ca²⁺, Sr²⁺, Ba²⁺, Cu⁺, Cu²⁺, Ag⁺, Zn²⁺, Mn²⁺, Fe²⁺, Fe³⁺, Y³⁺ und Ln³⁺, ist bzw. ist die anorganische, biokompatible Hülle daraus aufgebaut. Besonders bevorzugt ist das Metall (bzw. das Metallkation) aus der Gruppe, bestehend aus ZrO²⁺, Mg²⁺, Ca²⁺ und La³⁺, ausgewählt.

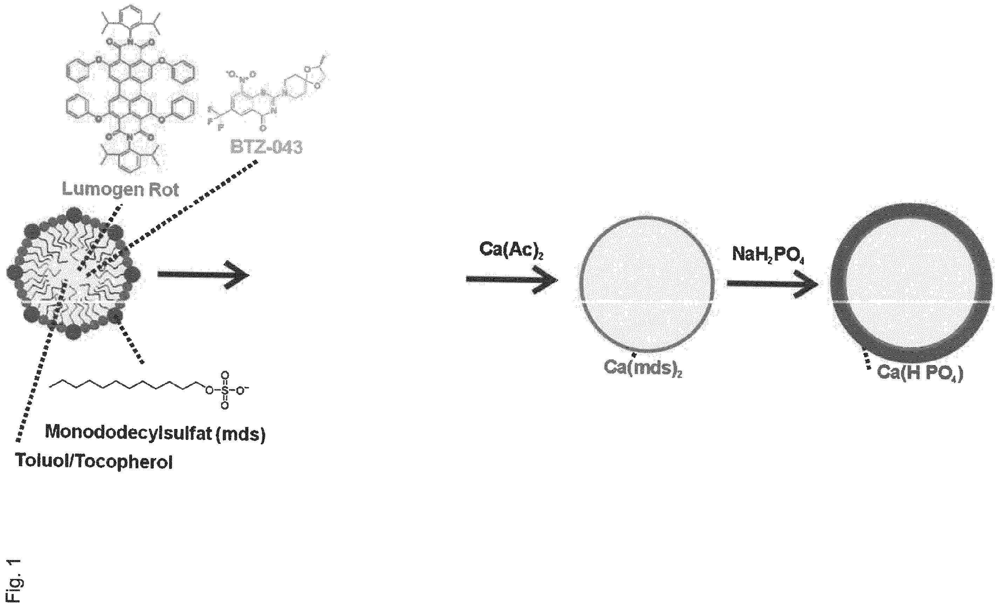

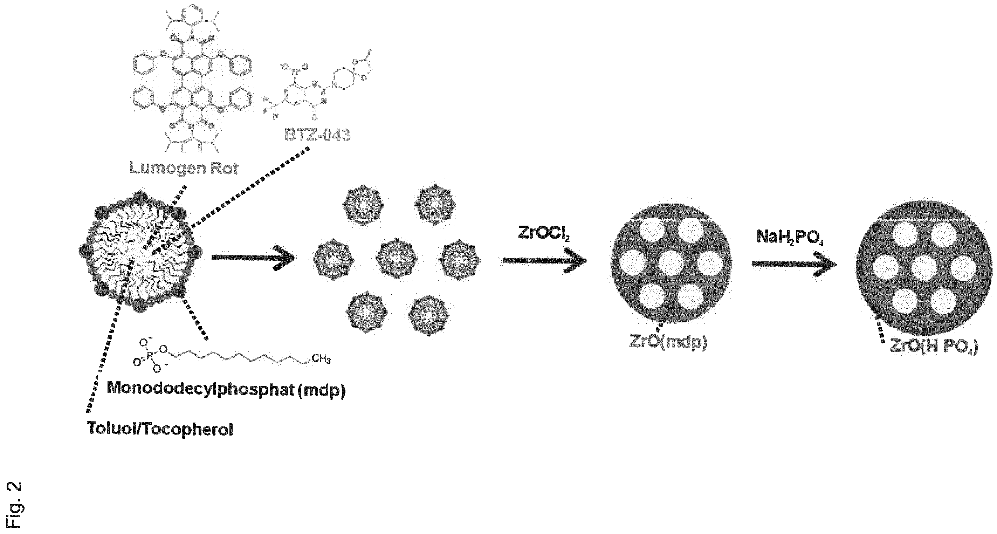

Gemäß einer Ausführungsform der vorliegenden Erfindung bildet die Emulsion mindestens eine Mizelle, wobei in der Mizelle das Tensid eine Schicht um die unpolare, lipophile Verbindung bildet und die polare Gruppe des Tensids ionisch an die anorganische, biokompatible Hülle gebunden ist (siehe Figur 1). Gemäß einer alternativen Ausführungsform bildet die Emulsion zwei oder mehr Mizellen, wobei in jeder der Mizellen das Tensid eine Schicht um die unpolare, lipophile Verbindung bildet. Jede der mindestens zwei Mizellen ist von einer anorganischen, biokompatiblen Hülle einzeln umschlossen, wobei die polaren Gruppen der Tenside jeder Mizelle ionisch an die anorganische, biokompatible Hülle gebunden sind. Zudem umschließt eine weitere anorganische, biokompatible Hülle die Agglomeration aus zwei oder mehr Mizellen gemeinsam (siehe Figur 2).

Gemäß der Ansprüche weist der erfindungsgemäße Nanocontainer einen Durchmesser von 10 bis 200 nm auf. Umfasst der Nanocontainer zwei oder mehr Mizellen wie in Figur 2, so weisen die jeweiligen Mizellen üblicherweise einen Durchmesser von 30 bis 100 nm auf.

Die Struktur um die unpolare, lipophile Verbindung in einem spezifischen Ausführungsbeispiel eines Nanocontainer der vorliegenden Erfindung lässt sich durch die allgemeine Formel [ZrO]²⁺[R(*Tensid*)OPO₃]²⁻@[ZrO]²⁺[HOPO₃]²⁻ als Kern@Schale-Struktur beschreiben, wobei der Rest R für den hydrophoben Tensidrest steht, und die polare Phosphatgruppe des Tensids ionisch an der inneren Oberfläche der Zirkonylphosphathülle eingebaut ist (siehe Figur 2).

Die vorliegende Erfindung betrifft ferner ein Verfahren gemäß Anspruch 9 zur Herstellung des erfindungsgemäßen Nanocontainers, umfassend die Schritte:
(a) Bereitstellen einer Emulsion, umfassend mindestens eine unpolare, lipophile Verbindung und ein biokompatibles Tensid, welches mindestens eine polare Gruppe, ausgewählt aus einer Phosphatgruppe, einer Sulfatgruppe, einer Sulfonatgruppe oder einer Carboxylgruppe, aufweist;
(b) Hinzufügen eines Metallsalzes, wobei die polaren Gruppen des Tensids durch Metallkationen des Metallsalzes unter Bildung einer schwerlöslichen Verbindung stabilisiert werden;
(c) Hinzufügen eines Phosphatsalzes, eines Hydrogenphosphatsalzes, eines Dihydrogenphosphatsalzes, eines Sulfatsalzes oder eines Carbonatsalzes zum Bilden einer anorganischen, biokompatiblen Hülle mit den Metallkationen aus Schritt (b) derart, dass die anorganische, biokompatible Hülle das gebildete Metallphosphat, Metallhydrogenphosphat, Metalldihydrogenphosphat, Metallsulfat oder Metallcarbonat umfasst;
wobei die anorganische, biokompatible Hülle die Emulsion umschließt und die polare Gruppe des biokompatiblen Tensids ionisch an die anorganische, biokompatible Hülle gebunden wird und dadurch das Tensid in Folge seiner Ausrichtung die Kavität der anorganischen Hülle lipophilisiert.

Sämtliche vorstehende Ausführungen bezüglich des erfindungsgemäßen Nanocontainers treffen auch für das erfindungsgemäße Verfahren zur Herstellung des Nanocontainers zu.

Im Schritt (a) des erfindungsgemäßen Verfahrens wird eine Emulsion bereitgestellt, umfassend eine unpolare, lipophile Verbindung und ein biokompatibles Tensid, welches mindestens eine polare Gruppe, ausgewählt aus einer Phosphatgruppe, einer Sulfatgruppe, einer Sulfonatgruppe oder einer Carboxylgruppe, aufweist. Im erfindungsgemäßen Verfahren fungiert das biokompatible Tensid als Reaktand. Als Lösungsmittel kann beispielsweise Wasser, isotones Wasser, ein physiologischer Puffer, ein Alkohol oder ein Gemisch aus mehreren dieser Lösungsmittel verwendet werden. Bevorzugte Alkohole zur Verwendung als Lösungsmittel sind Methanol, Ethanol, Propanol, Isopropanol und n-Butanol. Ferner kann diese wässrige Emulsion optional auch lipophile (Hilfs-)Lösungsmittel enthalten, beispielsweise Toluol und/oder Tocopherol. Gemäß einer weiteren Ausführungsform kann die wässrige Emulsion jedoch auch keine lipophilen (Hilfs-)Lösungsmittel enthalten, was dann die Beladungsmenge an unpolarer, lipophiler Verbindung pro Nanocontainer im Vergleich zu wässrigen Emulsionen, die ein lipophiles (Hilfs-)Lösungsmittel enthalten, stark erhöht (um ca. den Faktor 10 bis 50). Gemäß einer noch weiteren Ausführungsform kann die Emulsion einen pharmazeutisch aktiven Wirkstoff und ein Nachweisreagens als lipophile, unpolare Verbindungen umfassen.

Im Schritt (b) des erfindungsgemäßen Verfahrens wird der Emulsion aus Schritt (a) ein Metallsalz hinzugefügt, wodurch die polaren Gruppen des Tensids durch die Metallkationen des Metallsalzes unter Bildung einer schwerlöslichen Verbindung stabilisiert werden. Das Metallsalz kann sowohl als Feststoff als auch als Lösung oder Suspension hinzugefügt werden. Vorzugsweise werden ebenfalls die vorstehend genannten Lösungsmittel, nämlich Wasser, isotones Wasser, Alkohole sowie Gemische aus mehreren dieser Lösungsmittel verwendet, in denen das Metallsalz löslich ist. Das hinzugefügte Metallsalz (ein weiterer Reaktand des erfindungsgemäßen Verfahrens) wird dadurch dem ersten Reaktanden, dem biokompatiblen Tensid, nicht über getrennte Phasensysteme (polare/unpolare Flüssigphase), sondern im gleichen (polaren) Phasensystem zugefügt. Dadurch bildet sich eine Hülle ((Hohl)-Kugelwand) als schwerlösliche Verbindung durch Reaktion des Metallkations (in der wässrigen Dispersionsphase) mit dem biokompatiblen Tensid. Aufgrund der Verfahrensschritte (a) und (b) befindet sich das biokompatible Tensid nach der Reaktion auf der Innenseite der Hohlkugel, wird also eingekapselt (zusammen mit dem lipophilen Wirkstoff). Das Tensid kann nach der Reaktion nicht mehr abgetrennt werden kann. Es ist daher sehr wichtig, dass das Tensid biokompatibel ist.

In einer besonders bevorzugten Ausführungsform der vorliegenden Erfindung wird Wasser als Lösungsmittel verwendet, insbesondere Wasser, das auf einen pH-Wert von 4 bis 8 gepuffert ist, besonders bevorzugt auf einen pH-Wert von kleiner 7.

Die Metallkationen des Metallsalzes sind aus Zr⁴⁺, ZrO²⁺, Mg²⁺, Ca²⁺, Sr²⁺, Ba²⁺, Cu⁺, Cu²⁺, Ag⁺, Zn²⁺, Mn²⁺, Fe²⁺, Fe³⁺, Y³⁺ oder Ln³⁺ ausgewählt. Die Anionen des Metallsalzes unterliegen keiner besonderen Einschränkung. Bevorzugt werden Anionen verwendet, die mit den Metallkationen im verwendeten Lösungsmittel leichtlösliche Verbindungen bilden. Geeignete Metallsalze sind dem Fachmann bekannt. Vorzugsweise können als Metallsalze die Halogenide, Nitrate und Sulfate der vorstehend genannten Metalle verwendet werden. In einer besonders bevorzugten Ausführungsform der vorliegenden Erfindung wird Zirkonylchlorid als Metallsalz verwendet.

Die durch Metallkation-Tensid-Bindung im Schritt (b) aufgebaute Hohlkugelwand ist naturgemäß sehr dünn und damit chemisch/mechanisch nicht sehr stabil. Aus diesem Grund wird im Schritt (c) eine Verstärkung der Hohlkugelwand durchgeführt. Insbesondere wird im Schritt (c) des erfindungsgemäßen Verfahrens ein Phosphatsalz, ein Hydrogenphosphatsalz, eine Dihydrogenphosphatsalz, ein Sulfatsalz oder ein Carbonatsalz zum Bilden einer (sehr stabilen) anorganischen, biokompatiblen Hülle mit den Metallkationen aus Schritt (b) hinzugefügt, so dass die anorganische, biokompatible Hülle das gebildete Metallphosphat, Metallhydrogenphosphat, Metalldihydrogenphosphat, Metallsulfat oder Metallcarbonat umfasst bzw. daraus aufgebaut ist. Die anorganische, biokompatible Hülle umschließt die Emulsion und die polare Gruppe des biokompatiblen Tensids wird ionisch an die anorganische, biokompatible Hülle gebunden. Dadurch lipophilisiert das Tensid in Folge seiner Ausrichtung die Kavität der anorganischen Hülle. Die Reihenfolge der Schritte (b) und (c) im erfindungsgemäßen Verfahren ist nicht festgelegt, d.h. der Schritt (c) kann entweder vor oder nach dem Schritt (b) erfolgen, ohne dass sich dies auf den erhaltenen Nanocontainer auswirkt.

Das Phosphatsalz, Hydrogenphosphatsalz, Dihydrogenphosphatsalz, Sulfatsalz oder Carbonatsalz wird im Schritt (c) als Feststoff oder Lösung bzw. Suspension hinzugefügt. Vorzugsweise werden ebenfalls die vorstehend genannten Lösungsmittel, nämlich Wasser, isotones Wasser, Alkohole sowie Gemische aus mehreren dieser Lösungsmittel verwendet, in denen das Metallsalz löslich ist. Das Salz, ein weiterer Reaktand des erfindungsgemäßen Verfahrens, wird somit erneut ausschließlich über die Dispersionsphase (vorzugsweise Wasser) zugeführt. In einer besonders bevorzugten Ausführungsform der vorliegenden Erfindung wird daher Wasser als Lösungsmittel verwendet, insbesondere Wasser das auf einen pH-Wert von 4 bis 8 gepuffert ist, besonders bevorzugt auf einen pH-Wert von kleiner 7.

Im Schritt (b) und/oder im Schritt (c) kann optional ein Additiv zugegeben werden, um den pH-Wert zu senken bzw. diesen zu puffern. Beispiele für verwendbare Additive sind organische Säuren wie Zitronensäure, Oxalsäure, Weinsäure, Ameisensäure, Essigsäure usw. Gemäß einer bevorzugten Ausführungsform wird als Additiv Zitronensäure verwendet, insbesondere dann, wenn die polare Gruppe des biokompatiblen Tensids eine Sulfonatgruppe oder Sulfatgruppe aufweist.

Die Kationen des Phosphatsalzes, Hydrogenphosphatsalzes, Dihydrogenphosphatsalzes, Sulfatsalzes oder Carbonatsalzes unterliegen keiner besonderen Beschränkung. Vorzugsweise werden als Kationen Metalle eingesetzt, die zu leichtlöslichen Phosphaten, Hydrogenphosphaten, Dihydrogenphosphaten, Sulfaten oder Carbonaten führen. Gemäß einer bevorzugten Ausführungsform werden Natriumsalze verwendet, insbesondere Natriumdihydrogenphosphat. Gemäß einer alternativen Ausführungsform können auch Phosphate, Hydrogenphosphate, Dihydrogenphosphate, Sulfate oder Carbonate verwendet werden, die als Kation eines oder mehrere der für den Schritt (b) genannten Metallkationen aufweisen.

Die Reaktionstemperatur des erfindungsgemäßen Verfahrens unterliegt keiner besonderen Beschränkung. Vorzugsweise wird das Verfahren bei Raumtemperatur durchgeführt.

Nach Durchführung der Schritte (b) und (c) des erfindungsgemäßen Verfahrens fällt der entstandene (schwerlösliche) Nanocontainer aus bzw. ist im verwendeten Lösungsmittel suspendiert. An dieser Stelle des Verfahrens kann optional der Schritt (d) erfolgen.

Der optionale Schritt (d) des erfindungsgemäßen Verfahrens umfasst das Isolieren und/oder Aufreinigen des ausgefällten Nanocontainers. Dieses Isolieren und/oder Aufreinigen kann durch alle geeigneten Verfahren erfolgen. Derartige Verfahren sind im Stand der Technik bekannt.

Vorzugsweise erfolgt das Isolieren und/oder Aufreinigen der Hybridverbindungspartikel durch ein Verfahren, ausgewählt aus der Gruppe, bestehend aus Zentrifugationstechniken, Dialysetechniken, Phasentransfertechniken, Chromatographietechniken, Ultrafiltrationstechniken, Waschtechniken und Kombinationen davon. Die vorstehend genannten Verfahren zur Isolierung und/oder Aufreinigung der Hybridverbindungspartikel können auch kombiniert und/oder mehrfach ausgeführt werden.

Die vorliegende Erfindung betrifft ferner die Verwendung des erfindungsgemäßen Nanocontainers bzw. des über das erfindungsgemäße Verfahren hergestellten Nanocontainer in der Behandlung von Infektionen durch Bakterien und/oder Viren oder zur Behandlung von Tumoren. Insbesondere kann der Nanocontainer zur Behandlung von Infektionen durch Bakterien, wobei das Bakterium Tuberkulose verursacht oder Multiresistenzen aufweist, verwendet werden.

Zusammenfassend weist die vorliegende Erfindung folgende Vorteile gegenüber dem Stand der Technik auf:
- Nanocontainer als neuartiges Materialkonzept zur Verabreichung unpolarer, lipophiler Verbindungen wie Antibiotika;
- vorzugsweise enthält der Nanocontainer sowohl einen pharmazeutisch aktiven Wirkstoff für die Therapie als auch ein Nachweisreagens, wie beispielsweise einen Fluoreszenzfarbstoff für die Diagnostik;
- der Nanocontainer ist durch einfache optische Detektion über beispielsweise die Fluoreszenz eines enthaltenen Fluoreszenzfarbstoffanions in Zellen, Gewebe oder Organen möglich. Zusätzlich ist eine Detektion durch Röntgenabsorption oder magnetische Messungen möglich;
- die Synthese kann durch einfache Fällung in wässriger Phase erfolgen, da der Nanocontainer in Wasser schwerlöslich ist;
- der Nanocontainer kann sehr unterschiedliche unpolare, lipophile Verbindungen enthalten und erlaubt daher einen Einsatz für ein sehr breites medizinisches Therapiegebiet. Die unpolare, lipophile Verbindung wird aus dem Nanocontainer unter physiologischen Bedingungen zeitverzögert über einen Bereich von einigen Stunden bis zu einigen Tagen freigesetzt. Auf diesem Wege kann eine bestimmte Wirkstoffdosis über einen längeren Zeitraum und unmittelbar am Wirkort freigesetzt werden. Nebenwirkungen bzw. der unerwünschte Abbau des Wirkstoffs unter physiologischen Bedingungen, zum Beispiel im Blut, kann herabgesetzt bzw. vermieden werden;
- ebenso kann der Nanocontainer sehr unterschiedliche Nachweisreagenzien wie beispielsweise Fluoreszenzfarbstoffe enthalten. Typischerweise erfolgt die Anregung im Spektralbereich des sichtbaren Lichtes mit geeigneten Lasern oder Leuchtdioden (LEDs) unter Emission im Bereich des sichtbaren Lichtes bzw. im Infraroten;
- der Nanocontainer zeichnet sich neben der unpolaren, lipophilen Verbindung durch nicht-allergene bzw. nicht-toxische Bestandteile aus, die unter physiologischen Bedingungen vollständig abgebaut ausgeschieden werden.

Die Figuren zeigen:
Figur 1 eine Ausführungsform mit einer Mizelle: Einkapselung lipophiler Wirkstoffe in Calciummonododecylsulfat@Calciumhydrogenphosphat Nanocontainer am Beispiel der Füllung mit BTZ-043 (Antibiotikum) und Lumogen Rot (Fluoreszenzfarbstoff) sowie von Toluol und/oder Tocopherol als Lösungshilfsmittel; und
Figur 2 eine Ausführungsbeispiel mit mehreren Mizellen: Einkapselung lipophiler Wirkstoffe in Zirkonylmonododecylphosphat@Zirkonylhydrogenphosphat Nanocontainer am Beispiel der Füllung mit BTZ-043 (Antibiotikum) und Lumogen Rot (Fluoreszenzfarbstoff) sowie von Toluol und/oder Tocopherol als Lösungshilfsmittel.

Die vorliegende Erfindung wird durch die nachstehenden, nicht-beschränkenden Beispiele weiter erläutert.

### Beispiele:

### Ausführungsbeispiel 1:

### BTZ-043 in Zirkonylmonododecylphosphat@Zirkonylhydrogenphosphat-Nanocontainern

Es wurden 5mL deionisiertes Wasser, 500 mg (1,67 mmol) Natriummonododecylphosphat, 1,1 mL (12,02 mmol) n-Butanol, 0,5 mL (4,72 mmol) Toluol und 70 mg (0,16 mmol) BTZ-043 (2-(2-methyl-1,4-dioxa-8-azaspiro(4.5)dec-8-yl)-8-nitro-6-(trifluoromethyl)-4H-1,3-b enzothiazin-4-on) gemischt und mit 0,1 mL 1M Salzsäure versetzt. Durch kräftiges Rühren wurde nach etwa 10 Minuten eine transparente, stabile Emulsion erhalten. Je 0,5 mL der Emulsion wurden mittels eines Spritzenfilters filtriert und dann langsam (30 Minuten) zu je 20 mL einer wässrigen, Acetat-gepufferten Lösung von 84 mg (0,26 mmol) ZrOCl₂ × 8 H₂O mit einem pH-Wert von 4,7 getropft. Es wurde für eine Stunde gerührt und dann unter Kühlung in einem Eisbad 3,8 mL (0,38 mmol) einer wässrigen 0,1M NaH₂PO₄-Lösung innerhalb von einer Stunde zugetropft. Die Suspension wurde für weitere 20 Stunden gerührt, anschließend zwei Mal durch wiederholtes Abzentrifugieren und Resuspendieren in deionisiertem Wasser gewaschen und dann in 20 mL deionisiertem Wasser resuspendiert. Zur Bestimmung der Partikelkonzentration wurden zweimal je 1 mL der Suspension abpipettiert, in Aluminium-Wägeschälchen überführt und für 16 Stunden bei 95 °C im Trockenschrank getrocknet. Anschließend wurde das Trockengewicht bestimmt. Die Partikelkonzentration beträgt durchschnittlich 3,3 mg/mL. Durch Hinzufügen von Dextran-40 wurde schließlich eine Suspension in einer 5 Gew.-%igen, wässrigen Dextran-40-Lösung mit einer Partikelkonzentration von 3,3 mg/mL hergestellt. Die Bestimmung der Wirkstoffkonzentration bzw. -beladung erfolgte für Suspensionen mit einer Partikelkonzentration von 3,3 mg/mL mittels einer UV-VIS-Kalibriergeraden. Die BTZ-043-Konzentration liegt demnach bei 99 µg/mL.

### Ausführungsbeispiel 2:

### Bedaquilin in Zirkonylmonododecylphosphat@Zirkonylhydrogenphosphat Nanocontainern

Es wurden 5 mL deionisiertes Wasser, 500 mg (1,67 mmol) Natriummonododecylphosphat, 1,1 mL (12,02 mmol) n-Butanol, 0,5 mL (4,72 mmol) Toluol und 48 mg (0,09 mmol) Bedaquilin gemischt und mit 0,1 mL 1M Salzsäure versetzt. Durch kräftiges Rühren wurde nach etwa 10 Minuten eine transparente, stabile Emulsion erhalten. Je 0,5 mL der Emulsion wurden mittels eines Spritzenfilters filtriert und dann langsam (30 Minuten) zu je 20 mL einer wässrigen, Acetat-gepufferten Lösung von 84 mg (0,26 mmol) ZrOCl₂ × 8 H₂O mit einem pH-Wert von 4,7 getropft. Es wurde für eine Stunde gerührt und dann unter Kühlung in einem Eisbad 3,8 mL (0,38 mmol) einer wässrigen 0,1M NaH₂PO₄-Lösung innerhalb von einer Stunde zugetropft. Die Suspension wurde für weitere 20 Stunden gerührt, anschließend zwei Mal durch wiederholtes Abzentrifugieren und Resuspendieren in deionisiertem Wasser gewaschen und dann in 20 mL deionisiertem Wasser resuspendiert. Die Bestimmung von Partikel- und Wirkstoffkonzentration wurde durchgeführt wie in Ausführungsbeispiel 1 beschrieben und ergab eine Partikelkonzentration von 3,3 mg/mL in einer 5 Gew.-%igen, wässrigen Dextran-40-Lösung mit einer Bedaquilin-Beladung beträgt demnach 150 µg/mL.

### Ausführungsbeispiel 3:

### Clofazimin in Zirkonylmonododecylphosphat@Zirkonylhydrogenphosphat Nanocontainern

Es wurden 5 mL deionisiertes Wasser, 500 mg (1,67 mmol) Natriummonododecylphosphat, 1,1 mL (12,02 mmol) n-Butanol, 0,5 mL (4,72 mmol) Toluol und 36 mg (0,08 mmol) Clofazimin gemischt und mit 0,1 mL 1M Salzsäure versetzt. Durch kräftiges Rühren wurde nach etwa 10 Minuten eine transparente, stabile Emulsion erhalten. Je 0,5 mL der Emulsion wurden mittels eines Spritzenfilters filtriert und dann langsam (30 Minuten) zu je 20 mL einer wässrigen, Acetat-gepufferten Lösung von 84 mg (0,26 mmol) ZrOCl₂ × 8 H₂O mit einem pH-Wert von 4,7 getropft. Es wurde für eine Stunde gerührt und dann unter Kühlung in einem Eisbad 3,8 mL (0,38 mmol) einer wässrigen 0,1M NaH₂PO₄-Lösung innerhalb von einer Stunde zugetropft. Die Suspension wurde für weitere 20 Stunden gerührt, anschließend zwei Mal durch wiederholtes Abzentrifugieren und Resuspendieren in deionisiertem Wasser gewaschen und dann in 20 mL deionisiertem Wasser resuspendiert. Die Bestimmung von Partikel- und Wirkstoffkonzentration wurde durchgeführt wie in Ausführungsbeispiel 1 beschrieben und ergab eine Partikelkonzentration von 3,3 mg/mL in einer 5 Gew.-%igen, wässrigen Dextran-40-Lösung mit einer Clofazimin-Konzentration von 112 µg/mL.

### Ausführungsbeispiel 4:

### Lansoprazol in Zirkonylmonododecylphosphat@Zirkonylhydrogenphosphat Nanocontainern

Es wurden 5mL deionisiertes Wasser, 500 mg (1,67 mmol) Natriummonododecylphosphat, 1,1 mL (12,02 mmol) n-Butanol, 0,5 mL (4,72 mmol) Toluol und 30 mg (0,08 mmol) Lansoprazol gemischt und mit 0,1 mL 1M Salzsäure versetzt. Durch kräftiges Rühren wurde nach etwa 10 Minuten eine transparente, stabile Emulsion erhalten. Je 0,5 mL der Emulsion wurden mittels eines Spritzenfilters filtriert und dann langsam (30 Minuten) zu je 20 mL einer wässrigen, Acetat-gepufferten Lösung von 84 mg (0,26 mmol) ZrOCl₂ × 8 H₂O mit einem pH-Wert von 4,7 getropft. Es wurde für eine Stunde gerührt und dann unter Kühlung in einem Eisbad 3,8 mL (0,38 mmol) einer wässrigen 0,1M NaH₂PO₄-Lösung innerhalb von einer Stunde zugetropft. Die Suspension wurde für weitere 20 Stunden gerührt, anschließend zwei Mal durch wiederholtes Abzentrifugieren und Resuspendieren in deionisiertem Wasser gewaschen und dann in 20 mL deionisiertem Wasser resuspendiert. Die Bestimmung von Partikel- und Wirkstoffkonzentration wurde durchgeführt wie in Ausführungsbeispiel 1 beschrieben und ergab eine Partikelkonzentration von 3,3 mg/mL in einer 5 Gew.-%igen, wässrigen Dextran-40-Lösung mit einer Lansoprazol-Beladung beträgt demnach 155 µg/mL.

### Ausführungsbeispiel 5:

### BTZ-043 und Lumogen Rot in Zirkonylmonododecylphosphat@Zirkonylhydrogenphosphat Nanocontainern

Es wurden 5 mL deionisiertes Wasser, 500 mg (1,67 mmol) Natriummonododecylphosphat, 1,1 mL (12,02 mmol) n-Butanol, 0,5 mL (4,72 mmol) Toluol, 70 mg (0,16 mmol) BTZ-043 und 2 mg (1,9 µmol) Lumogen Rot gemischt und mit 0,1 mL 1M Salzsäure versetzt. Durch kräftiges Rühren wurde nach etwa 10 Minuten eine transparente, stabile Emulsion erhalten. Je 0,5 mL der Emulsion wurden mittels eines Spritzenfilters filtriert und dann langsam (30 Minuten) zu je 20 mL einer wässrigen, Acetat-gepufferten Lösung von 84 mg (0,26 mmol) ZrOCl₂ × 8 H₂O mit einem pH-Wert von 4,7 getropft. Es wurde für eine Stunde gerührt und dann unter Kühlung in einem Eisbad 3,8 mL (0,38 mmol) einer wässrigen 0,1M NaH₂PO₄-Lösung innerhalb von einer Stunde zugetropft. Die Suspension wurde für weitere 20 Stunden gerührt, anschließend zwei Mal durch wiederholtes Abzentrifugieren und Resuspendieren in deionisiertem Wasser gewaschen und dann in 20 mL deionisiertem Wasser resuspendiert. Die Bestimmung von Partikel- und Wirkstoffkonzentration wurde durchgeführt wie in Ausführungsbeispiel 1 beschrieben und ergab eine Partikelkonzentration von 3,3 mg/mL in einer 5 Gew.-%igen, wässrigen Dextran-40-Lösung mit einer BTZ-043-Beladung von 99 µg/mL und einer Lumogen Rot-Beladung von 3 µg/mL.

### Ausführungsbeispiel 6:

### Bedaquilin und Lumogen Rot in Zirkonylmonododecylphosphat@Zirkonylhydrogenphosphat Nanocontainern

Es wurden 5 mL deionisiertes Wasser, 500 mg (1,67 mmol) Natriummonododecylphosphat, 1,1 mL (12,02 mmol) n-Butanol, 0,5 mL (4,72 mmol) Toluol, 48 mg (0,09 mmol) Bedaquilin und 2 mg (1,9 µmol) Lumogen Rot gemischt und mit 0,1 mL 1M Salzsäure versetzt. Durch kräftiges Rühren wurde nach etwa 10 Minuten eine transparente, stabile Emulsion erhalten. Je 0,5 mL der Emulsion wurden mittels eines Spritzenfilters filtriert und dann langsam (30 Minuten) zu je 20 mL einer wässrigen, Acetat-gepufferten Lösung von 84 mg (0,26 mmol) ZrOCl₂ × 8 H₂O mit einem pH-Wert von 4,7 getropft. Es wurde für eine Stunde gerührt und dann unter Kühlung in einem Eisbad 3,8 mL (0,38 mmol) einer wässrigen 0,1M NaH₂PO₄-Lösung innerhalb von einer Stunde zugetropft. Die Suspension wurde für weitere 20 Stunden gerührt, anschließend zwei Mal durch wiederholtes Abzentrifugieren und Resuspendieren in deionisiertem Wasser gewaschen und dann in 20 mL deionisiertem Wasser resuspendiert. Die Bestimmung von Partikel- und Wirkstoffkonzentration wurde durchgeführt wie in Ausführungsbeispiel 1 beschrieben und ergab eine Partikelkonzentration von 3,3 mg/mL in einer 5 Gew.-%igen, wässrigen Dextran-40-Lösung mit einer Bedaquilin-Beladung beträgt demnach 144 µg/mL und diejenige für Lumogen Rot 3 µg/mL.

### Ausführungsbeispiel 7:

### Irinotecan in Zirkonylmonododecylphosphat@Zirkonylhydrogenphosphat Nanocontainern

Es wurden 5 mL deionisiertes Wasser, 500 mg (1,67 mmol) Natriummonododecylphosphat, 1,1 mL (12,02 mmol) n-Butanol, 0,5 mL (4,72 mmol) Toluol und 35 mg (0,06 mmol) Irinotecan gemischt und mit 0,1 mL 1M Salzsäure versetzt. Durch kräftiges Rühren wurde nach etwa 10 Minuten eine transparente, stabile Emulsion erhalten. Je 0,5 mL der Emulsion wurden mittels eines Spritzenfilters filtriert und dann langsam (30 Minuten) zu je 20 mL einer wässrigen, Acetat-gepufferten Lösung von 84 mg (0,26 mmol) ZrOCl₂ × 8 H₂O mit einem pH-Wert von 4,7 getropft. Es wurde für eine Stunde gerührt und dann unter Kühlung in einem Eisbad 3,8 mL (0,38 mmol) einer wässrigen 0,1M NaH₂PO₄-Lösung innerhalb von einer Stunde zugetropft. Die Suspension wurde für weitere 20 Stunden gerührt, anschließend zwei Mal durch wiederholtes Abzentrifugieren und Resuspendieren in deionisiertem Wasser gewaschen und dann in 20 mL deionisiertem Wasser resuspendiert. Die Bestimmung von Partikel- und Wirkstoffkonzentration wurde durchgeführt wie in Ausführungsbeispiel 1 beschrieben und ergab eine Partikelkonzentration von 3,3 mg/mL in einer 5 Gew.-%igen, wässrigen Dextran-40-Lösung mit einer Irinotecan-Beladung beträgt demnach 87 µg/mL.

### Ausführungsbeispiel 8:

### Paclitaxel in Zirkonylmonododecylphosphat@Zirkonylhydrogenphosphat Nanocontainern

Es wurden 5 mL deionisiertes Wasser, 500 mg (1,67 mmol) Natriummonododecylphosphat, 1,1 mL (12,02 mmol) n-Butanol, 0,5 mL (4,72 mmol) Toluol und 1 mg (1,2 µmol) Paclitaxel gemischt und mit 0,1 mL 1M Salzsäure versetzt. Durch kräftiges Rühren wurde nach etwa 10 Minuten eine transparente, stabile Emulsion erhalten. Je 0,5 mL der Emulsion wurden mittels eines Spritzenfilters filtriert und dann langsam (30 Minuten) zu je 20 mL einer wässrigen, Acetat-gepufferten Lösung von 84 mg (0,26 mmol) ZrOCl₂ × 8 H₂O mit einem pH-Wert von 4,7 getropft. Es wurde für eine Stunde gerührt und dann unter Kühlung in einem Eisbad 3,8 mL (0,38 mmol) einer wässrigen 0,1M NaH₂PO₄-Lösung innerhalb von einer Stunde zugetropft. Die Suspension wurde für weitere 20 Stunden gerührt, anschließend zwei Mal durch wiederholtes Abzentrifugieren und Resuspendieren in deionisiertem Wasser gewaschen und dann in 20 mL deionisiertem Wasser resuspendiert. Die Bestimmung von Partikel- und Wirkstoffkonzentration wurde durchgeführt wie in Ausführungsbeispiel 1 beschrieben und ergab eine Partikelkonzentration von 3,3 mg/mL in einer 5 Gew.-%igen, wässrigen Dextran-40-Lösung mit einer Paclitaxel-Beladung beträgt demnach 12 µg/mL.

### Ausführungsbeispiel 9:

### BTZ-043 in Zirkonylmonododecylphosphat@Zirkonylhydrogenphosphat Nanocontainern

Es wurden 5 mL deionisiertes Wasser, 500 mg (1,67 mmol) Natriummonododecylphosphat, 1,1 mL (12,02 mmol) *n*-Butanol und 700 mg (1,6 mmol) BTZ-043 gemischt und mit 0,1 mL 1M Salzsäure versetzt. Durch kräftiges Rühren wurde nach etwa 10 Minuten eine transparente, stabile Emulsion erhalten. Je 0,5 mL der Emulsion wurden mittels eines Spritzenfilters filtriert und dann langsam (30 Minuten) zu je 20 mL einer wässrigen, Acetat-gepufferten Lösung von 84 mg (0,26 mmol) ZrOCl₂ × 8 H₂O mit einem pH-Wert von 4,7 getropft. Es wurde für eine Stunde gerührt und dann unter Kühlung in einem Eisbad 3,8 mL (0,38 mmol) einer wässrigen 0,1M NaH₂PO₄-Lösung innerhalb von einer Stunde zugetropft. Die Suspension wurde für weitere 20 Stunden gerührt, anschließend zwei Mal durch wiederholtes Abzentrifugieren und Resuspendieren in deionisiertem Wasser gewaschen und dann in 20 mL deionisiertem Wasser resuspendiert. Die Bestimmung von Partikel- und Wirkstoffkonzentration wurde durchgeführt wie in Ausführungsbeispiel 1 beschrieben und ergab eine Partikelkonzentration von 3,3 mg/mL in einer 5 Gew.-%igen, wässrigen Dextran-40-Lösung mit einer BTZ-43-Beladung beträgt demnach 990 µg/mL.

### Ausführungsbeispiel 10:

### Clofazimin in Zirkonylmonododecylphosphat@Zirkonylhydrogenphosphat Nanocontainern

Es wurden 5 mL deionisiertes Wasser, 500 mg (1,67 mmol) Natriummonododecylphosphat, 1,1 mL (12,02 mmol) n-Butanol, und 360 mg (0,8 mmol) Clofazimin gemischt und mit 0,1 mL 1M Salzsäure versetzt. Durch kräftiges Rühren wurde nach etwa 10 Minuten eine transparente, stabile Emulsion erhalten. Je 0,5 mL der Emulsion wurden mittels eines Spritzenfilters filtriert und dann langsam (30 Minuten) zu je 20 mL einer wässrigen, Acetat-gepufferten Lösung von 84 mg (0,26 mmol) ZrOCl₂ × 8 H₂O mit einem pH-Wert von 4,7 getropft. Es wurde für eine Stunde gerührt und dann unter Kühlung in einem Eisbad 3,8 mL (0,38 mmol) einer wässrigen 0,1M NaH₂PO₄-Lösung innerhalb von einer Stunde zugetropft. Die Suspension wurde für weitere 20 Stunden gerührt, anschließend zwei Mal durch wiederholtes Abzentrifugieren und Resuspendieren in deionisiertem Wasser gewaschen und dann in 20 mL deionisiertem Wasser resuspendiert. Die Bestimmung von Partikel- und Wirkstoffkonzentration wurde durchgeführt wie in Ausführungsbeispiel 1 beschrieben und ergab eine Partikelkonzentration von 3,3 mg/mL in einer 5 Gew.-%igen, wässrigen Dextran-40-Lösung mit einer Clofazimin-Konzentration von 1120 µg/mL.

### Ausführungsbeispiel 11:

### Irinotecan in Zirkonylmonododecylphosphat@Zirkonylhydrogenphosphat Nanocontainern

Es wurden 5 mL deionisiertes Wasser, 500 mg (1,67 mmol) Natriummonododecylphosphat, 1,1 mL (12,02 mmol) n-Butanol und 350 mg (0,6 mmol) Irinotecan gemischt und mit 0,1 mL 1M Salzsäure versetzt. Durch kräftiges Rühren wurde nach etwa 10 Minuten eine transparente, stabile Emulsion erhalten. Je 0,5 mL der Emulsion wurden mittels eines Spritzenfilters filtriert und dann langsam (30 Minuten) zu je 20 mL einer wässrigen, Acetat-gepufferten Lösung von 84 mg (0,26 mmol) ZrOCl₂ × 8 H₂O mit einem pH-Wert von 4,7 getropft. Es wurde für eine Stunde gerührt und dann unter Kühlung in einem Eisbad 3,8 mL (0,38 mmol) einer wässrigen 0,1M NaH₂PO₄-Lösung innerhalb von einer Stunde zugetropft. Die Suspension wurde für weitere 20 Stunden gerührt, anschließend zwei Mal durch wiederholtes Abzentrifugieren und Resuspendieren in deionisiertem Wasser gewaschen und dann in 20 mL deionisiertem Wasser resuspendiert. Die Bestimmung von Partikel- und Wirkstoffkonzentration wurde durchgeführt wie in Ausführungsbeispiel 1 beschrieben und ergab eine Partikelkonzentration von 3,3 mg/mL in einer 5 Gew.-%igen, wässrigen Dextran-40-Lösung mit einer Irinotecan-Beladung beträgt demnach 870 µg/mL.

### Ausführungsbeispiel 12:

### BTZ-043 in Calciumdodecylsulfat@Calciumhydrogenphosphat Nanocontainern

Eine gelbe transparente Emulsion wurde durch Mischen von 2,5 ml destilliertem Wasser (140 mmol), 700 mg SDS (2,43 mmol), 0,45 ml Tocopherol (0,43 g, 0,99 mmol), 26 mg (0,07 mmol) BTZ-043 und 0,45 ml Butanol (0,36 g, 4,91 mmol) erhalten, das Gesamtvolumen betrug 3,25 ml. Zu Beginn der Synthese wurden 0,2 ml der zuvor hergestellten Mikroemulsion, entsprechend 43 mg SDS (0,149 mmol, 1 Äq.), 1,6 mg (0,004 mmol) BTZ-043 und 27.6 µl Tocopherol (26,31 mg, 0,0601 mmol), zu einer Lösung von 221 mg Na₂HPO₄ (1,24 mmol, 8,3 Äq.) und 33 mg Zitronensäure (0,15 mmol) in 50 ml Wasser gegeben. Die Lösung wurde mit 0,5 M NaOH auf pH 6,1 gebracht und 338 mg Ca-Acetat (2,13 mmol, 14,2 Äq.) in 5 ml Wasser tropfenweise zugegeben. Die Suspension wurde für weitere 20 Stunden gerührt, anschließend zwei Mal durch wiederholtes Abzentrifugieren und Resuspendieren in deionisiertem Wasser gewaschen und dann in deionisiertem Wasser resuspendiert. Die Bestimmung von Partikel- und Wirkstoffkonzentration wurde durchgeführt wie in Ausführungsbeispiel 1 beschrieben und ergab eine Partikelkonzentration von 3,3 mg/mL in einer 5 Gew.-%igen, wässrigen Dextran-40-Lösung mit einer BTZ-Beladung beträgt demnach 40 µg/mL.

### Ausführungsbeispiel 13:

### BTZ-043 in Calciumdodecylsulfat@Calciumhydrogenphosphat Nanocontainern

Eine gelbe transparente Emulsion wurde durch Mischen von 2,5 ml destilliertem Wasser (140 mmol), 700 mg SDS (2,43 mmol), 260 mg (0,7 mmol) BTZ-043 und 0,45 ml Butanol (0,36 g, 4,91 mmol) erhalten, das Gesamtvolumen betrug 3,25 ml. Zu Beginn der Synthese wurden 0,2 ml der zuvor hergestellten Mikroemulsion, entsprechend 43 mg SDS (0,149 mmol, 1 Äq.) und 16 mg (0,04 mmol) BTZ-043 zu einer Lösung von 221 mg Na₂HPO₄ (1,24 mmol, 8,3 Äq.) und 33 mg Zitronensäure (0,15 mmol) in 50 ml Wasser gegeben. Die Lösung wurde mit 0,5 M NaOH auf pH 6,1 gebracht und 338 mg Ca-Acetat (2,13 mmol, 14,2 Äq.) in 5 ml Wasser tropfenweise zugegeben. Die Suspension wurde für weitere 20 Stunden gerührt, anschließend zwei Mal durch wiederholtes Abzentrifugieren und Resuspendieren in deionisiertem Wasser gewaschen und dann in deionisiertem Wasser resuspendiert. Die Bestimmung von Partikel- und Wirkstoffkonzentration wurde durchgeführt wie in Ausführungsbeispiel 1 beschrieben und ergab eine Partikelkonzentration von 3,3 mg/mL in einer 5 Gew.-%igen, wässrigen Dextran-40-Lösung mit einer BTZ-Beladung beträgt demnach 400 µg/mL.

### Ausführungsbeispiel 14:

### BTZ-043 in Lanthanlaurylsulfat/-hydrogenphosphat-Nanocontainern

Es wurde eine Lösung von 30 mL deionisiertem Wasser, 15 mg (0,05 mmol) Natriumlaurylsulfat und 90 mg (1,1 mmol) Ammoniumacetat hergestellt und mit einem Eisbad gekühlt. Nachfolgend wurde eine Lösung mit 40 mg (0,3 mmol) BTZ043 in 1,2 ml Dimethylsulfoxid (DMSO) unter starkem Rühren zugespritzt. Während der Zugabe erfolgte die Durchmischung der Flüssigphase zusätzlich durch Ultraschallpulse von 10 Sekunden Dauer. Nachfolgend wurden langsam (30 Minuten) 19 mg (0,05 mmol) LaCl₃×7H₂O gelöst in 1,0 mL Wasser bei einem pH-Wert von 7,0 zugetropft. Es wurde für eine Stunde gerührt und dann unter Kühlung in einem Eisbad 0,5 mL (0,1 mmol) einer wässrigen 0,1M NaH₂PO₄-Lösung innerhalb von einer Stunde zugetropft. Die Suspension wurde für weitere 5 Stunden gerührt, anschließend zwei Mal durch wiederholtes Abzentrifugieren und Resuspendieren in deionisiertem Wasser gewaschen und dann in 10 mL deionisiertem Wasser resuspendiert. Es resultiert eine leicht gelbliche Suspension. Die Bestimmung von Partikel- und Wirkstoffkonzentration wurde durchgeführt wie in Ausführungsbeispiel 1 beschrieben und ergab eine Partikelgröße um 40 nm und einer Wirkstoffkonzentration von 4 mg/ml BTZ.

### Ausführungsbeispiel 15:

### BTZ-043 in Zirkonylmonododecylphosphat/-hydrogenphosphat-Nanocontainern

Es wurde eine Lösung von 30 mL deionisiertem Wasser, 25 mg (0,08 mmol) Natriummonododecylphosphat und 90 mg (1,1 mmol) Ammoniumacetat hergestellt und mit einem Eisbad gekühlt. Nachfolgend wurde eine Lösung mit 40 mg (0,3 mmol) BTZ043 in 1,2 ml Dimethylsulfoxid (DMSO) unter starkem Rühren zugespritzt. Während der Zugabe erfolgte die Durchmischung der Flüssigphase zusätzlich durch Ultraschallpulse von 10 Sekunden Dauer. Nachfolgend werden langsam (30 Minuten) 26 mg (0,08 mmol) ZrOCl₂×8H₂O gelöst in 1,0 mL Wasser bei einem pH-Wert von 7,0 zugetropft. Es wurde für eine Stunde gerührt und dann unter Kühlung in einem Eisbad 1,0 mL (0,1 mmol) einer wässrigen 0,1M NaH₂PO₄-Lösung innerhalb von einer Stunde zugetropft. Die Suspension wurde für weitere 5 Stunden gerührt, anschließend zwei Mal durch wiederholtes Abzentrifugieren und Resuspendieren in deionisiertem Wasser gewaschen und dann in 10 mL deionisiertem Wasser resuspendiert. Es resultiert eine leicht gelbliche Suspension Die Bestimmung von Partikel- und Wirkstoffkonzentration wurde durchgeführt wie in Ausführungsbeispiel 1 beschrieben und ergab eine Partikelgröße um 40 nm und einer Wirkstoffkonzentration von 4 mg/ml BTZ.

### Ausführungsbeispiel 16:

### Bedaquilin in Zirkonylmonododecylphosphat/-hydrogenphosphat-Nanocontainern

3,6 mg Natriummonododecylphosphat (0.01 mmol) wurden mit 2 Tropfen Butanol und 1 ml deionisiertem Wasser bis zur vollständigen Auflösung bei 50 °C gerührt. 2,5 mg Tocopherolphosphat (0.005 mmol) wurden hinzugegeben und gerührt bis zur vollständigen Auflösung. Nachfolgend wurde eine Lösung mit 30 mg Ammoniumacetat (0.4 mmol) in 5 ml Wasser hinzugegeben, und das resultierende Gemisch in einem Eisbad gekühlt. Nachfolgend wurde eine Lösung mit 10 mg (0,02 mmol) Bedaquiline in 0,4 ml Dimethylsulfoxid (DMSO) unter starkem Rühren zugespritzt. Während der Zugabe erfolgte die Durchmischung der Flüssigphase zusätzlich durch Ultraschallpulse von 10 Sekunden Dauer. Nachfolgend wurden langsam (30 Minuten) 3 mg (0,01 mmol) FeCl₃×6H₂O gelöst in 1,0 mL Wasser bei einem pH-Wert von 7,0 zugetropft. Es wurde für eine Stunde gerührt und dann unter Kühlung in einem Eisbad 0,2 mL (0,02 mmol) einer wässrigen 0,1 M NaH₂PO₄-Lösung mit 3,9 mg 3-Phosphopropionsäure innerhalb von einer Stunde zugetropft. Die Suspension wurde für weitere 5 Stunden gerührt, anschließend zwei Mal durch wiederholtes Abzentrifugieren und Resuspendieren in deionisiertem Wasser gewaschen und dann in 2 mL deionisiertem Wasser resuspendiert. Es resultiert eine leicht gelbliche Suspension. Die Bestimmung von Partikel- und Wirkstoffkonzentration wurde durchgeführt wie in Ausführungsbeispiel 1 beschrieben und ergab eine Partikelgröße um 20 nm und einer Wirkstoffkonzentration von 5 mg/ml Bedaquilin.

## Patentansprüche

1. Nanocontainer, umfassend
eine Emulsion, umfassend mindestens eine unpolare, lipophile Verbindung, eingekapselt von einem biokompatiblen Tensid, welches mindestens eine polare Gruppe, ausgewählt aus einer Phosphatgruppe, einer Sulfatgruppe, einer Sulfonatgruppe oder einer Carboxylgruppe, aufweist, und
eine anorganische, biokompatible Hülle, welche die Emulsion umschließt, wobei die anorganische, biokompatible Hülle ein Metallphosphat, ein Metallhydrogenphosphat, ein Metalldihydrogenphosphat, ein Metallsulfat oder ein Metallcarbonat umfasst, wobei das Metall mindestens ein Metall, ausgewählt aus der Gruppe, bestehend aus Zr⁴⁺, ZrO²⁺, Mg²⁺, Ca²⁺, Sr²⁺, Ba²⁺, Cu⁺, Cu²⁺, Ag⁺, Zn²⁺, Mn²⁺, Fe²⁺, Fe³⁺, Y³⁺ und Ln³⁺, vorzugsweise ein Metall, ausgewählt aus der Gruppe, bestehend aus ZrO²⁺, Mg²⁺, Ca²⁺ und La³⁺, ist,
wobei die polare Gruppe ionisch an die anorganische, biokompatible Hülle gebunden ist und dadurch das Tensid in Folge seiner Ausrichtung die Kavität der anorganischen Hülle lipophilisiert,
wobei der Nanocontainer einen Durchmesser von 10 bis 200 nm aufweist, und
wobei die mindestens eine unpolare, lipophile Verbindung ein pharmazeutisch aktiver Wirkstoff und/oder ein Nachweisreagens ist.

2. Nanocontainer nach Anspruch 1, wobei der pharmazeutisch aktive Wirkstoff bzw. das Nachweisreagens ausgewählt sind aus
der Gruppe der Antibiotika, bestehend aus Delamanid, Bedaquilin, Benzothiazon, Amikazin, Clofazimin, Levofloxacin, Ofloxacin, Rifampicin, Pantoprazol, Pyrimethamin, Trimethoprim, Sulfamethoxazol, Sulfadoxin, Novobiocin, Coumermycin, Clorobiocin, Metronidazol, Norfloxacin, Enoxacin, Ciprofloxacin, Ofloxacin, Levofloxacin, Moxifloxacin, Tigecyclin, Tetracyclin, Gentamicin, Kanamycin, Neomycin, Netilmicin, Streptomycin, Tobramycin,
Chloramphenicol, Fusidinsäure, Cethromycin, Narbomycin, Telithromycin, Lincomycin, Daptomycin, Dalfopristin, Quinupristin, Azithromycin, Clarithromycin, Erythromycin, Roxithromycin, Linezolid, Doxycyclin, Minocyclin, Tetracyclin, Oxytetracyclin, Tigecyclin Imipenem, Meropenem, Ertapenem, Aztreonam, Benzylpenicillin, Phenoxymethylpenicillin, Piperacillin, Mezlocillin, Ampicillin, Amoxicillin, Flucloxacillin, Methicillin, Oxacillin, Clavulansäure, Sulbactam, Tazobactam, Sultamicillin, Teicoplanin, Vancomycin, Bacitracin, Colistin, Gramicidin, Polymyxin B, Tyrothricin und Teixobactin; oder
aus der Gruppe der Cytostatika, bestehend aus Cyclophosphamid, Mechlorethamin, Dacarbazine, Nitrosoureas, Temozolomid, Daunorubicin, Epirubicin, Idarubicin, Mitoxantron, Valrubicin, Paclitaxel, Docetaxel, Abraxan, Taxoter, Vorinostat, Romidepsin, Irinotecan, Topotecan, Etoposid, Teniposid, Tafluposid, Bortezomib, Erlotinib, Gefitinib, Imatinib, Vemurafenib, Vismodegib, Azacitidin, Azathioprin, Capecitabin, Cytarabin, Doxifluridin, Fluorouracil, Gemcitabin, Hydroxyurea, Vinblastin, Vincristin, Vindesin und Vinorelbine; oder
aus der Gruppe der Virostatika, bestehend aus Ancriviroc, Aplaviroc, Cenicriviroc, Enfuvirtid, Maraviroc, Vicriviroc, Amantadin, Rimantadin, Pleconaril, Idoxuridin, Aciclovir, Brivudin, Famciclovir, Penciclovir, Sorivudin, Valaciclovir, Cidofovir, Brincidofovir, Ganciclovir, Valganciclovir, Foscarnet, Ribavirin, Taribavirin, Filibuvir, Nesbuvir, Sofosbuvir, Tegobuvir, Favipiravir, Abacavir, Didanosin, Elvucitabin, Emtricitabin, Fosalvudintidoxil, Fozivudintidoxil, Stavudin, Zalcitabin, Zidovudin, Lamivudin, Lagociclovir, Tenofovir, Adefovir, Alamifovir, Clevudin, Entecavir, Pradefovir, Telbivudin, Delavirdin, Efavirenz, Emivirin, Etravirin, Lersivirin, Nevirapin, Rilpivirin, Amprenavir, Atazanavir, Brecanavir, Darunavir, Fosamprenavir, Indinavir, Lopinavir, Mozenavir, Nelfinavir, Ritonavir, Saquinavir, Tipranavir, Asunaprevir, Balapiravir, Boceprevir, Ciluprevir, Danoprevir, Daclatasvir, Narlaprevir, Telaprevir, Simeprevir, Vaniprevir, Rupintrivir, Elvitegravir, Dolutegravir, Raltegravir, Fomivirsen, Amenamevir, Bevirimat, Letermovir, Laninamivir, Oseltamivir, Peramivir und Zanamivir; oder
aus der Gruppe der Fluoreszenzfarbstoffe, bestehend aus Coumarin 6, Lumogen Rot, Fluoresceindiacetat, Oxonol, Nilrot und Fluoresceinisothiocyanat.

3. Nanocontainer nach Anspruch 1 oder 2, wobei die Masse eines pharmazeutisch aktiven Wirkstoffs mindestens 5 Gew.-%, bezogen auf die Gesamtmasse des Nanocontainers, beträgt.

4. Nanocontainer nach einem der Ansprüche 1 bis 3, wobei das Tensid ein Alkylphosphat, ein Alkylsulfat, ein Alkylsulfonat oder ein Alkylcarboxylat umfasst.

5. Nanocontainer nach Anspruch 4, wobei das Tensid Natrium- oder Kaliummonodecylphosphat, Natrium- oder Kaliumdodecylphosphat, Natrium- oder Kaliumdodecylsulfat, Natrium- oder Kaliumlaurinat oder Natrium- oder Kaliumcaprinat umfasst.

6. Nanocontainer nach einem der Ansprüche 1 bis 5, wobei die anorganische, biokompatible Hülle ein Zeta-Potential bei pH 7 von mindestens -20 mV aufweist.

7. Nanocontainer nach einem der Ansprüche 1 bis 6, wobei die Emulsion mindestens eine Mizelle bildet, wobei in der mindestens einen Mizelle das Tensid eine Schicht um die unpolare, lipophile Verbindung bildet und die polare Gruppe des Tensids ionisch an die anorganische, biokompatible Hülle gebunden ist.

8. Nanocontainer nach einem der Ansprüche 1 bis 7, wobei eine Struktur um die unpolare, lipophile Verbindung [ZrO]²⁺[R(*Tensid*)OPO₃]²⁻ ist, wobei der Rest R für den hydrophoben Tensidrest steht, und die polare Phosphatgruppe des Tensids ionisch in die Zirkonylphosphathülle eingebaut ist.

9. Verfahren zur Herstellung eines Nanocontainers nach einem der Ansprüche 1 bis 8, umfassend die Schritte:
(a) Bereitstellen einer Emulsion, umfassend mindestens eine unpolare, lipophile Verbindung und ein biokompatibles Tensid, welches mindestens eine polare Gruppe, ausgewählt aus einer Phosphatgruppe, einer Sulfatgruppe, einer Sulfonatgruppe oder einer Carboxylgruppe, aufweist;
(b) Hinzufügen eines Metallsalzes, wobei die polaren Gruppen des Tensids durch Metallkationen des Metallsalzes unter Bildung einer schwerlöslichen Verbindung stabilisiert werden, wobei das Metall mindestens ein Metall, ausgewählt aus der Gruppe, bestehend aus Zr⁴⁺, ZrO²⁺, Mg²⁺, Ca²⁺, Sr²⁺, Ba²⁺, Cu⁺, Cu²⁺, Ag⁺, Zn²⁺, Mn²⁺, Fe²⁺, Fe³⁺, Y³⁺ und Ln³⁺, vorzugsweise ein Metall, ausgewählt aus der Gruppe, bestehend aus ZrO²⁺, Mg²⁺, Ca²⁺ und La³⁺, ist;
(c) Hinzufügen eines Phosphatsalzes, eines Hydrogenphosphatsalzes, eines Dihydrogenphosphatsalzes, eines Sulfatsalzes oder eines Carbonatsalzes zum Bilden einer anorganischen, biokompatiblen Hülle mit den Metallkationen aus Schritt (b) derart, dass die anorganische, biokompatible Hülle das gebildete Metallphosphat, Metallhydrogenphosphat, Metalldihydrogenphosphat, Metallsulfat oder Metallcarbonat umfasst;
wobei die anorganische, biokompatible Hülle die Emulsion umschließt und die polare Gruppe des biokompatiblen Tensids ionisch an die anorganische, biokompatible Hülle gebunden wird und dadurch das Tensid in Folge seiner Ausrichtung die Kavität der anorganischen Hülle lipophilisiert.

10. Nanocontainer nach einem der Ansprüche 1 bis 8 zur Verwendung in der Behandlung von Infektionen durch Bakterien und/oder Viren oder zur Behandlung von Tumoren.

11. Nanocontainer zur Verwendung nach Anspruch 10, wobei das Bakterium Tuberkulose verursacht oder Multiresistenzen aufweist.

## Claims

1. Nanocontainer, comprising
an emulsion comprising at least one nonpolar, lipophilic compound encapsulated by a biocompatible surfactant having at least one polar group selected from a phosphate group, a sulfate group, a sulfonate group or a carboxyl group, and
an inorganic, biocompatible shell enclosing the emulsion, wherein the inorganic, biocompatible shell comprises a metal phosphate, a metal hydrogen phosphate, a metal dihydrogen phosphate, a metal sulfate, or a metal carbonate, wherein the metal is at least one metal selected from the group consisting of Zr⁴⁺, ZrO²⁺, Mg²⁺, Ca²⁺, Sr²⁺, Ba²⁺, Cu⁺, Cu²⁺, Ag⁺, Zn²⁺, Mn²⁺, Fe²⁺, Fe³⁺, Y³⁺, and Ln³⁺, preferably a metal selected from the group consisting of ZrO²⁺, Mg ²⁺, Ca²⁺, and La³⁺,
wherein the polar group is ionically bound to the inorganic, biocompatible shell and, as a result of its orientation, the surfactant lipophilizes the cavity of the inorganic shell,
wherein the nanocontainer has a diameter of 10 to 200 nm , and
wherein at least one nonpolar, lipophilic compound is a pharmaceutically active drug and/or a detection reagent.

2. Nanocontainer according to claim 1, wherein the pharmaceutically active substance or the detection reagent is selected from
the group of antibiotics consisting of delamanid, bedaquiline, benzothiazone, amikazin, clofazimine, levofloxacin, ofloxacin, rifampicin, pantoprazole, pyrimethamine, trimethoprim, sulfamethoxazole, sulfadoxine, novobiocin, coumermycin, clorobiocin, metronidazole, norfloxacin, enoxacin, ciprofloxacin, ofloxacin, levofloxacin, moxifloxacin, tigecycline, tetracycline, gentamicin, kanamycin, neomycin, netilmicin, streptomycin, tobramycin, chloramphenicol, fusidic acid, cethromycin, narbomycin, telithromycin, lincomycin, daptomycin, dalfopristin, quinupristin, azithromycin, clarithromycin, erythromycin, roxithromycin, linezolid, doxycycline, minocycline, tetracycline, oxytetracycline, tigecycline, imipenem, meropenem, ertapenem, aztreonam, benzylpenicillin, phenoxymethylpenicillin, piperacillin, mezlocillin, ampicillin, amoxicillin,
flucloxacillin, methicillin, oxacillin, clavulanic acid, sulbactam, tazobactam, sultamicillin, teicoplanin, vancomycin, bacitracin, colistin, gramicidin, polymyxin B, tyrothricin and teixobactin; or
from the group of cytostatic drugs, consisting of cyclophosphamide, mechlorethamine, dacarbazines, nitrosoureas, temozolomide, daunorubicin, epirubicin, idarubicin, mitoxantrone, valrubicin, paclitaxel, docetaxel, abraxan, taxoter, vorinostat, romidepsin, irinotecan, topotecan, etoposide, teniposide, tafluposide, bortezomib, erlotinib, gefitinib, imatinib, vemurafenib, vismodegib, azacitidine, azathioprine, capecitabine, cytarabine, doxifluridine, fluorouracil, gemcitabine, hydroxyurea, vinblastine, vincristine, vindesine, and vinorelbine; or
from the group of antivirals, consisting of ancriviroc, aplaviroc, cenicriviroc , enfuvirtide, maraviroc, vicriviroc, amantadine, rimantadine, pleconaril, idoxuridine, acyclovir, brivudine, famciclovir, penciclovir, sorivudine, valaciclovir, cidofovir, brincidofovir, ganciclovir, valganciclovir, foscarnet, ribavirin, taribavirin, filibuvir, nesbuvir, sofosbuvir, tegobuvir, favipiravir, abacavir, didanosine, elvucitabine, emtricitabine, fosalvudintidoxil, fozivudintidoxil, stavudine, zalcitabine, zidovudine, lamivudine, lagociclovir, tenofovir, adefovir, alamifovir, clevudine, entecavir, pradefovir, telbivudine, delavirdine, efavirenz, emivirine, etravirine, lersivirine, nevirapine, rilpivirine, amprenavir, atazanavir, brecanavir, darunavir, fosamprenavir, indinavir, iopinavir, mozenavir, nelfinavir, ritonavir, saquinavir, tipranavir, asunaprevir, balapiravir, oceprevir, ciluprevir, danoprevir, daclatasvir, narlaprevir, telaprevir, simeprevir, vaniprevir, rupintrivir, elvitegravir, dolutegravir, raltegravir, fomivirsen, amenamevir, bevirimat, letermovir, laninamivir, oseltamivir, peramivir and zanamivir; or
from the group of fluorescent dyes, consisting of coumarin 6, lumigen red, fluorescein diacetate, oxonol, nile red and fluorescein isothiocyanate.

3. Nanocontainer according to claim 1 or 2, wherein the mass of a pharmaceutically active ingredient is at least 5 wt-%, based on the total mass of the nanocontainer.

4. Nanocontainer according to any one of claims 1 to 3, wherein the surfactant comprises an alkyl phosphate, an alkyl sulfate, an alkyl sulfonate or an alkyl carboxylate .

5. Nanocontainer according to claim 4, wherein the surfactant comprises sodium or potassium monodecyl phosphate, sodium or potassium dodecyl phosphate, sodium or potassium dodecyl sulfate, sodium or potassium laurinate or sodium or potassium caprinate.

6. Nanocontainer according to any one of claims 1 to 5, wherein the inorganic, biocompatible shell has a zeta potential at pH 7 of at least -20 mV.

7. Nanocontainer according to any one of claims 1 to 6, wherein the emulsion forms at least one micelle, wherein in the at least one micelle the surfactant forms a layer around the nonpolar, lipophilic compound and the polar group of the surfactant is ionically bound to the inorganic, biocompatible shell.

8. Nanocontainer according to one of claims 1 to 7, wherein a structure is formed around the nonpolar, lipophilic compound [ZrO]²⁺[R(*surfactant*)OPO_{3]}²⁻, wherein the residue R represents the hydrophobic surfactant residue, and the polar phosphate group of the surfactant is ionically incorporated into the zirconyl phosphate shell.

9. Method for manufacturing a nanocontainer according to any one of claims 1 to 8, comprising the steps:
(a) providing an emulsion comprising at least one nonpolar, lipophilic compound and a biocompatible surfactant having at least one polar group selected from a phosphate group, a sulfate group, a sulfonate group or a carboxyl group;
(b) adding a metal salt, wherein the polar groups of the surfactant are stabilized by metal cations of the metal salt to form a sparingly soluble compound, wherein the metal is at least a metal selected from the group consisting of Zr⁴⁺, ZrO²⁺, Mg²⁺, Ca²⁺, Sr²⁺, Ba²⁺, Cu⁺, Cu²⁺, Ag⁺, Zn²⁺, Mn²⁺, Fe²⁺, Fe³⁺, Y³⁺, and Ln³⁺, preferably a metal selected from the group consisting of ZrO²⁺, Mg²⁺, Ca²⁺, and La³⁺;
(c) adding a phosphate salt, a hydrogen phosphate salt, a dihydrogen phosphate salt, a sulfate salt or a carbonate salt to form an inorganic, biocompatible shell with the metal cations from step (b) such that the inorganic, biocompatible shell comprises the metal phosphate, metal hydrogen phosphate, metal dihydrogen phosphate, metal sulfate or metal carbonate formed;
wherein the inorganic, biocompatible shell encloses the emulsion and the polar group of the biocompatible surfactant is ionically bound to the inorganic, biocompatible shell, and thereby the surfactant, as a result of its orientation, lipophilizes the cavity of the inorganic shell.

10. Nanocontainer according to any one of claims 1 to 8 for use in the treatment of infections caused by bacteria and/or viruses or for the treatment of tumors.

11. Nanocontainer for use according to claim 10, wherein the bacterium causes tuberculosis or exhibits multi-resistance.

## Revendications

1. Nanoconteneur, comprenant
une émulsion comprenant au moins un composé lipophile non polaire, encapsulé par un tensioactif biocompatible qui présente au moins un groupe polaire sélectionné parmi un groupe phosphate, un groupe sulfate, un groupe sulfonate ou un groupe carboxyle, et
une enveloppe inorganique biocompatible qui renferme l'émulsion, dans lequel l'enveloppe inorganique biocompatible comprend un phosphate métallique, un hydrogénophosphate métallique, un dihydrogénophosphate métallique, un sulfate métallique ou un carbonate métallique, dans lequel le métal est au moins un métal sélectionné parmi le groupe constitué de Zr⁴⁺, ZrO²⁺, Mg²⁺, Ca²⁺, Sr²⁺, Ba²⁺, Cu⁺, Cu²⁺, Ag⁺, Zn²⁺, Mn²⁺, Fe²⁺, Fe³⁺, Y³⁺ et Ln³⁺, de préférence un métal sélectionné parmi le groupe constitué de ZrO²⁺, Mg²⁺, Ca²⁺, et La³⁺,
dans lequel le groupe polaire est lié de manière ionique à l'enveloppe inorganique biocompatible et le tensioactif lipophilise de ce fait la cavité de l'enveloppe inorganique à la suite de son orientation,
dans lequel le nanoconteneur présente un diamètre de 10 à 200 nm, et
dans lequel l'au moins un composé lipophile non polaire est un ingrédient pharmaceutiquement actif et/ou un réactif de détection.

2. Nanoconteneur selon la revendication 1, dans lequel l'ingrédient pharmaceutiquement actif et/ou le réactif de détection sont sélectionnés parmi
le groupe des antibiotiques, constitué du délamanide, bédaquiline, benzothiazone, amikacine, clofazimine, lévofloxacine, ofloxacine, rifampicine, pantoprazole, pyriméthamine, triméthoprime, sulfaméthoxazole, sulfadoxine, novobiocine, coumermycine, clorobiocine, métronidazole, norfloxacine, énoxacine, ciprofloxacine, ofloxacine, lévofloxacine, moxifloxacine, tigécycline, tétracycline, gentamicine, kanamycine, néomycine, nétilmicine, streptomycine, tobramycine, chloramphénicol, acide fusidique, céthromycine, narbomycine, télithromycine, lincomycine, daptomycine, dalfopristine, quinupristine, azithromycine, clarithromycine, érythromycine, roxithromycine, linézolide, doxycycline, minocycline, tétracycline, oxytétracycline, tigécycline, imipénem, méropénem, ertapénem, aztréonam, benzylpénicilline, phénoxyméthylpénicilline, pipéracilline, mezlocilline, ampicilline, amoxicilline, flucloxacilline, méthicilline, oxacilline, acide clavulanique, sulbactam, tazobactam, sultamicilline, téicoplanine, vancomycine, bacitracine, colistine, gramidicine, polymyxine B, tyrothricine et teixobactine ; ou
parmi le groupe des agents cytostatiques, constitué du cyclophosphamide, méchloréthamine, dacarbazine, nitrosourées, témozolomide, daunorubicine, épirubicine, idarubicine, mitoxantrone, valrubicine, paclitaxel, docétaxel, abraxane, taxotère, vorinostat, romidepsine, irinotécan, topotécan, étoposide, téniposide, tafluposide, bortézomib, erlotinib, géfitinib, imatinib, vémurafénib, vismodégib, azacitidine, azothioprine, capécitabine, cytarabine, doxifluridine, fluorouracile, gemcitabine, hydroxyurée, vinblastine, vincristine, vindésine et vinorelbine ; ou
parmi le groupe des agents virostatiques, constitué de l'ancriviroc, aplaviroc, cénicriviroc, enfuvirtide, maraviroc, vicriviroc, amantadine, rimantadine, pléconaril, idoxuridine, aciclovir, brivudine, famciclovir, penciclovir, sorivudine, valaciclovir, cidofovir, brincidofovir, ganciclovir, valganciclovir, foscarnet, ribavirine, taribavirine, filibuvir, nesbuvir, sofosbuvir, tégobuvir, favipiravir, abacavir, didanosine, elvucitabine, emtricitabine, fosalvudintidoxil, fozivudintidoxil, stavudine, zalcitabine, zidovudine, lamivudine, lagociclovir, ténofovir, adéfovir, alamifovir, clévudine, entécavir, pradéfovir, telbivudine, délavirdine, éfavirenz, émivirine, étravirine, lersivirine, névirapine, rilpivirine, amprénavir, atazanavir, brécanavir, darunavir, fosamprénavir, indinavir, lopinavir, mozénavir, nelfinavir, ritonavir, saquinavir, tipranavir, asunaprévir, balapiravir, bocéprévir, ciluprévir, danoprévir, daclatasvir, narlaprévir, télaprévir, siméprévir, vaniprévir, rupintrivir, elvitégravir, dolutégravir, raltégravir, fomivirsen, aménamévir, bévirimat, létermovir, laninamivir, oseltamivir, péramivir et zanamivir ; ou
parmi le groupe des colorants fluorescents, constitué de la coumarine 6, du rouge Lumogen, du diacétate de fluorescéine, de l'oxonol, du rouge du Nil et de l'isothiocyanate de fluorescéine.

3. Nanoconteneur selon la revendication 1 ou 2, dans lequel la masse d'un ingrédient pharmaceutiquement actif se monte à au moins 5 % en poids par rapport à la masse totale du nanoconteneur.

4. Nanoconteneur selon une des revendications 1 à 3, dans lequel le tensioactif comprend un alkylphosphate, un alkylsulfate, un alkylsulfonate ou un alkylcarboxylate.

5. Nanoconteneur selon la revendication 4, dans lequel le tensioactif comprend du monodécylphosphate de sodium ou potassium, du dodécylphosphate de sodium ou potassium, du dodécylsulfate de sodium ou potassium, du laurinate de sodium ou potassium, ou du caprinate de sodium ou potassium.

6. Nanoconteneur selon une des revendications 1 à 5, dans lequel l'enveloppe inorganique biocompatible présente un potentiel zêta à pH 7 d'au moins -20 mV.

7. Nanoconteneur selon une des revendications 1 à 6, dans lequel l'émulsion forme au moins une micelle, dans lequel le tensioactif forme une couche autour du composé lipophile non polaire dans l'au moins une micelle et le groupe polaire du tensioactif est lié de manière ionique à l'enveloppe inorganique biocompatible.

8. Nanoconteneur selon une des revendications 1 à 7, dans lequel une structure autour du composé lipophile non polaire est [ZrO]²⁺[R(*Tensioactif*)OPO₃]²⁻*,* dans lequel le radical R représente le radical de tensioactif hydrophobe, et le groupe phosphate polaire du tensioactif est incorporé de manière ionique dans l'enveloppe de phosphate de zirconyle.

9. Procédé de fabrication d'un nanoconteneur selon une des revendications 1 à 8, comprenant les étapes :
(a) mise à disposition d'une émulsion comprenant au moins un composé lipophile non polaire et un tensioactif biocompatible qui présente au moins un groupe polaire sélectionné parmi un groupe phosphate, un groupe sulfate, un groupe sulfonate ou un groupe carboxyle ;
(b) ajout d'un sel métallique, dans lequel les groupes polaires du tensioactif sont stabilisés par des cations métalliques du sel métallique avec formation d'un composé difficilement soluble, dans lequel le métal est au moins un métal sélectionné parmi le groupe constitué de Zr⁴⁺, ZrO²⁺, Mg²⁺, Ca²⁺, Sr²⁺, Ba²⁺, Cu⁺, Cu²⁺, Ag⁺, Zn²⁺, Mn²⁺, Fe²⁺, Fe³⁺, Y³⁺ et Ln³⁺, de préférence un métal sélectionné parmi le groupe constitué de ZrO²⁺, Mg²⁺, Ca²⁺, et La³⁺ ;
(c) ajout d'un sel de phosphate, d'un sel d'hydrogénophosphate, d'un sel de dihydrogénophosphate, d'un sel de sulfate ou d'un sel de carbonate pour la formation d'une enveloppe inorganique biocompatible avec les cations métalliques de l'étape (b) de telle sorte que l'enveloppe inorganique biocompatible comprend le phosphate métallique, l'hydrogénophosphate métallique, le dihydrogénophosphate métallique, le sulfate métallique ou le carbonate métallique formé ;
dans lequel l'enveloppe inorganique biocompatible renferme l'émulsion et le groupe polaire du tensioactif biocompatible est lié de manière ionique à l'enveloppe inorganique biocompatible, et le tensioactif lipophilise de ce fait la cavité de l'enveloppe inorganique à la suite de son orientation.

10. Nanoconteneur selon une des revendications 1 à 8 destiné à l'utilisation dans le traitement d'infections par des bactéries et/ou virus ou destiné au traitement de tumeurs.

11. Nanoconteneur destiné à l'utilisation selon la revendication 10, dans lequel la bactérie provoque la tuberculose ou présente des multirésistances.
